**Europäisches Patentamt**

**European Patent Office**

**Office européen des brevets**

(19)

(11) Publication number : **0 440 658 B1**

# EUROPEAN PATENT SPECIFICATION

(12)

(45) Date of publication of patent specification :
**15.07.92 Bulletin 92/29**

(51) Int. Cl.⁵ : **A61M 16/00**

(21) Application number : **89908529.4**

(22) Date of filing : **26.07.89**

(86) International application number :
**PCT/NL89/00062**

(87) International publication number :
**WO 90/00910 08.02.90 Gazette 90/04**

(54) **A MOUTH-TO-MOUTH RESUSCITATION MASK.**

(30) Priority : **27.07.88 NL 8801887**

(43) Date of publication of application :
**14.08.91 Bulletin 91/33**

(45) Publication of the grant of the patent :
**15.07.92 Bulletin 92/29**

(84) Designated Contracting States :
**AT BE CH DE FR GB IT LI LU NL SE**

(56) References cited :
**AU-B- 448 073**
**DE-A- 2 742 213**
**DE-U- 8 801 386**
**US-A- 3 802 428**

(73) Proprietor : **Daniels, Robertus Franciscus Cornelia Maria**
**Nassaustraat 32**
**NL-5682 AD Best (NL)**

(72) Inventor : **Daniels, Robertus Franciscus Cornelia Maria**
**Nassaustraat 32**
**NL-5682 AD Best (NL)**

(74) Representative : **Smulders, Theodorus A.H.J., Ir. et al**
**Vereenigde Octrooibureaux Nieuwe Parklaan 97**
**NL-2587 BN 's-Gravenhage (NL)**

Jouve, 18, rue Saint-Denis, 75001 PARIS

EP 0 440 658 B1

## Description

This invention relates to a mouth-to-mouth resuscitation mask comprising a foil of flexible synthetic plastics material, provided with a thickened portion containing a hole provided with an air-permeable material, the thickened portion has the shape and size of a credit card.

It is an object of the present invention to improve such a mask disclosed in German Gebrauchsmuster 88 01 386, especially with reference to an easy use.

To that effect, the mask is characterized in that the foil is made of non-transparent material, preferably in surgical green or blood red, and is provided with a transparent eye window. This allows the mask to be carried conveniently and be added to the ordinary personal papers that are conventional or necessary in modern society. As the mask is provided with transparent eye holes, the patient's eyes may be observed during the treatment.

In further elaboration of the invention the foil may be provided circumferentially with an elastic edge and the gauzy material may be made in such a manner that air flows more easily from the outside inwards than the other way round, so that the mask can also serve to remedy hyperventilation problems: because the mask substantially seals over the face, the exhalation of too much air or nitrogen, producing the oppressed feeling in the event of hyperventilation, is prevented.

By impregnating the air-permeable portion with a scent, the use of the mask for mouth-to-mouth resuscitation can be promoted in a simple manner.

The gauzy material can be made in such a manner that air will flow more easily from the outside inwards than the other way round. As a result, it is not only possible to put the mask and hence the air-permeable portion on the victim's head in a more or less fixed position, but it is moreover achieved that the mask can in an excellent way serve to remedy hyperventilation.

In this simple manner, there is provided a life-saving aid that is easily carried along and can be used for both mouth-to-mouth resuscitation and to remedy hyperventilation symptoms.

For the sake of completeness it is stated that from GE-A-2,742,213 in itself is known to use a mouth-to-mouth resuscitation mask which is red coloured.

One embodiment of the mask according to the present invention will now be described, by way of example, with reference to the accompanying drawings, in which:

Fig. 1 is a front view of a mask according to the present invention worn on a head illustrated in stylized fashion;

Fig. 2 is a cross-sectional view taken on the line II-II of Fig. 1;

Fig. 3 shows on a reduced scale the mask according to Figs. 1 and 2 in folded condition; and

Fig. 4 is a cross-sectional view, on an enlarged scale, on the line IV-IV of Fig. 1.

As shown in the drawings, a mask is provided with a flexible foil 1 of synthetic plastics material wherein, at the location of the mouth of a head 2 shown in dash-dotted lines, there is provided a credit card-like, stiffened portion 3. The centre of stiffened portion 3 is provided with a hole 4 filled with a filler 7 covered on either side by a cover 5 of synthetic plastics material having apertures 8 therein. A suitable filter material is the material marketed by the company of Carl Freudenberg at Weinheim, BRD, under the name of Viledon Micro donfilter, type LRS 306.

Cover 5 can be made of synthetic plastics material, fine muslin, mull, gauze or the like: on the one hand a good air-permeability is required and on the other minimal contact between the victim and the donor. In order to render this aid "more agreable", said filter can be prepared with a scent, e.g. menthol, so that the donor is not troubled, or hardly so, by a possible unpleasant odour from the victim.

In this connection, it is observed that the foil can be coloured, so as to render any blood present on the victim inconspicuous, e.g. surgical green or blood red. In order that the victim's eyes may be observed, a transparent eye hole 9 is provided in the mask.

In order that the mask may also be used in the event the victim is suffering from hyperventilation, foil 1 may be provided with an elastic circumferential edge 6, so that the air or nitrogen exhaled by the victim remains underneath the mask, thereby preventing over-exhalation of air or nitrogen, producing the oppressed feeling or hyperventilation.

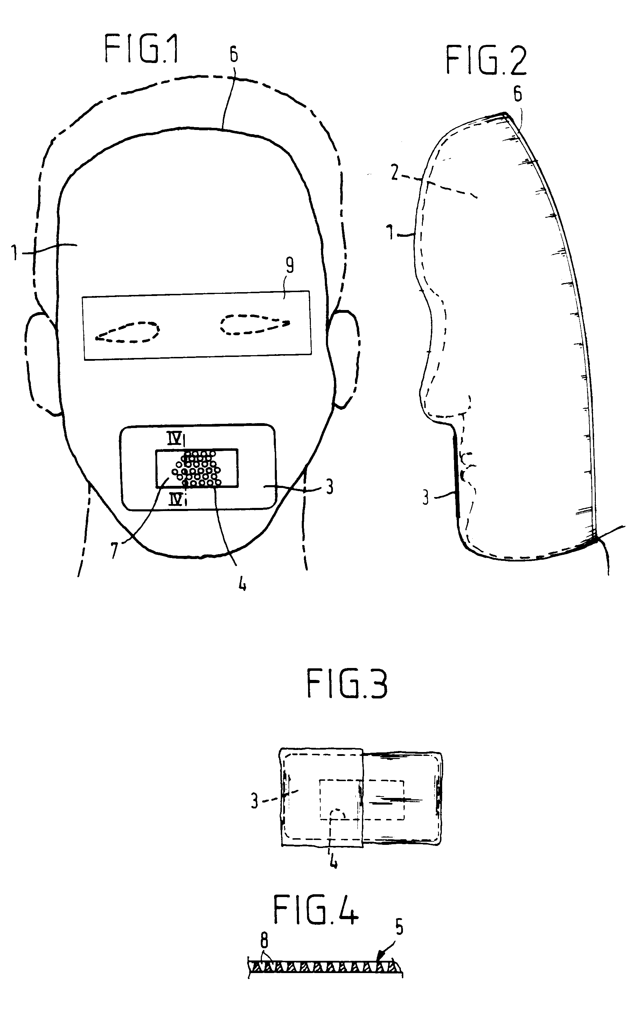

The illustration of the mask in folded condition in Fig. 3 appears to be self-explanatory. Possibly, the unit may be stored in a case carrying the bearer's personal data, such as name, address, blood group etc.

As noted above, the gauzy portion 5 should be made in such a manner that air or oxygen can be easily supplied therethrough to the victim, but on the other hand, this gauzy portion should be made in such a manner that the air cannot be easily discharged by the victim, in which case the contemplated effect in remedying hyperventilation would not occur. Preferably, the gauzy portion is made in such a manner that the air-permeability from the outside inwards is relatively high, but the air-permeability from the inside outwards is lower. This can be achieved e.g. by providing passages with a tapered configuration from the outside inwards: an example is shown in Fig. 4.

## Claims

1. A mouth-to-mouth resuscitation mask comprising a foil (1) of flexible synthetic plastics material, provided with a thickened portion (3) containing a hole (4) provided with an air-permeable material (7), the thickened portion has the shape and size of a credit card, characterized in that the foil is made of non-transparent material, preferably in surgical green or blood red, and is provided with a transparent eye window (9).

2. A mask as claimed in claim 1, characterized in that the foil is provided circumferentially with an elastic edge (6) and the gauzy material (7) is made in such a manner that air flows more easily from the outside inwards than the other way round (fig. 4).

3. A mask as claimed in claim 1 or 2, characterized in that the air-permeable material is impregnated with a scent, e.g. menthol.

4. A mask as claimed in claim 3, characterized in that the air passages are tapered (fig. 4) from the outside inwards.

5. A mask as claimed in any of the preceding claims, characterized in that it is packaged in a case on the outside of which the bearer's personal data can be written or displayed otherwise.

**Patentansprüche**

1. Mund-zu-Mund-Beatmungsmaske enthaltend eine Folie (1) aus flexiblem synthetischem Kunststoff, die mit einem verdickten Bereich (3) versehen ist, der eine mit einem luftdurchlässigen Material (7) versehene Öffnung (4) aufweist und die Form und Größe einer Kreditkarte besitzt, dadurch gekennzeichnet, daß die Folie aus einem undurchsichtigen Material, vorzugsweise in chirurgischem Grün oder Blutrot hergestellt ist und mit einem durchsichtigen Augenfenster (9) versehen ist.

2. Maske nach Anspruch 1, dadurch gekennzeichnet, daß die Folie (1) um den Umfang herum mit einem elastischen Saum (6) versehen ist und daß das gazeartige Material (7) derart beschaffen ist, daß die Luft leichter von außen nach innen strömt als umgekehrt (Fig. 4).

3. Maske nach Anspruch 1 oder 2, dadurch gekennzeichnet, daß das luftdurchlässige Material mit einem Duftstoff, z.B. Menthol, imprägniert ist.

4. Maske nach Anspruch 3, dadurch gekennzeichnet, daß sich die Luftöffnungen von außen nach innen hin verjüngen (Fig. 4).

5. Maske nach einem der vorherigen Ansprüche, dadurch gekennzeichnet, daß sie in eine Hülle verpackt ist, auf deren Außenseite die Personaldaten des Trägers geschrieben oder sonstwie angezeigt sein können.

**Revendications**

1. Masque de réanimation par bouche-à-bouche comprenant une feuille (1) en matière plastique synthétique flexible pourvue d'une partie épaissie (3) qui présente un trou (4) muni d'un matériau perméable à l'air (7), la forme et la taille de la partie épaissie étant celles d'une carte de crédit, caractérisé par le fait que la feuille est constituée par une matière non transparente, de préférence d'un vert chirurgical ou rouge sang, et qu'elle est pourvue d'une fenêtre transparente (9) pour les yeux.

2. Masque selon la revendication 1, caractérisé par le fait que la feuille est munie sur son pourtour d'un bord élastique (6), et que la matière en gaze (7) est réalisée d'une manière telle que l'air s'écoule plus facilement de l'extérieur vers l'intérieur que dans l'autre sens (figure 4).

3. Masque selon la revendication 1 ou 2, caractérisé par le fait que le matériau perméable à l'air est imprégné d'une substance odoriférante, par exemple de menthol.

4. Masque selon la revendication 3, caractérisé par le fait que les passages destinés à l'air se rétrécissent (figure 4) de l'extérieur vers l'intérieur.

5. Masque selon l'une quelconque des revendications précédentes, caractérisé par le fait qu'il est enveloppé dans un étui sur l'extérieur duquel les données personnelles du porteur peuvent être écrites ou affichées d'une autre manière.

# FIG.1

# FIG.2

# FIG.3

# FIG.4